# EUROPEAN PATENT APPLICATION

(11) **EP 4 718 364 A1**
(43) Date of publication of application: **01.04.2026**
(21) Application number: 25205176.8
(22) Date of filing: 29.09.2025
(51) Int. Cl.: G06Q 40/08, B60W 40/09

(54) **COMPUTE SYSTEM WITH RISK MANAGEMENT MECHANISM AND METHOD OF OPERATION THEREOF**

(30) Priority: 30.09.2024 US 202418902336
(71) Applicant: TeleNav, Inc., Santa Clara, CA 95054 (US)
(72) Inventor: Aist, Gregory Stewart, Santa Clara, CA, 95054 (US); Carter, Casey Thomas, Santa Clara, CA, 95054 (US)
(74) Representative: Lucke, Andreas

(57) **Abstract**

A compute system (100) includes: a control unit (113) configured to: analyze telematics data (114) of a first device (102) operated by a probational member (105); receive biometric data (116) of the probational member (105); identify map data (112) based on the telematics data (114), the biometric data (116), or a combination thereof; compile a probational member status (109) based on the telematics data (114), biometric data (116), the map data (112), or a combination thereof concurrently compiled by real-time monitoring a the probational member (105); facilitate a sponsoring member (118) and a member (120) of a shared risk pool (122) to access the probational member status (109); and generate a determination of a vote (312) by the sponsoring member (118) and the member (120) for an admission of the probational member (105) to the shared risk pool (122) based on the probational member status (109).

## Description

### TECHNICAL FIELD

An embodiment of the present invention relates generally to a compute system, and more particularly to a system with a risk management system.

### BACKGROUND ART

As the population continues to grow, costs as part of risk for individuals have increased exponentially. Corporations or other groups can combine individual groups or employee groups in order to distribute risk and keep costs under control. Individuals who might want to recommend risk management solutions to their friends lack any incentive to choose safer individuals or healthy individuals as the people to whom they recommend to the risk management company. Risk management companies spend a portion of their income on risk assessment for individuals in order to set premium levels. Statistics can generalize the safety or health levels of groups delineated by age or gender, but is ineffective to evaluate any individual. Whether the individual is applying for health or automobile risk management policy, the risk management company makes an educated guess based on generalized statistics.

Thus, a need still remains for a compute system with a risk management mechanism to provide a reduction of costs. In view of the ever-increasing commercial competitive pressures, along with growing consumer expectations and the diminishing opportunities for meaningful product differentiation in the marketplace, it is increasingly critical that answers be found to these problems. Additionally, the need to reduce costs, improve efficiencies and performance, and meet competitive pressures adds an even greater urgency to the critical necessity for finding answers to these problems.

Solutions to these problems have been long sought but prior developments have not taught or suggested any solutions and, thus, solutions to these problems have long eluded those skilled in the art.

### DISCLOSURE OF THE INVENTION

An embodiment of the present invention provides a method of operation of a compute system including analyzing telematics data of a first device operated by a probational member; receiving biometric data of the probational member; identifying map data based on the telematics data, the biometric data, or a combination thereof; compiling a probational member status based on the telematics data, biometric data, the map data, or a combination thereof concurrently compiled by real-time monitoring a the probational member; facilitating a sponsoring member and a member of a shared risk pool to access the probational member status; and generating a determination of a vote by the sponsoring member and the member for an admission of the probational member to the shared risk pool based on the probational member status.

The probational member status may be concurrently compiled by compiling information obtained concurrently through real-time monitoring the probational member and/or the first device. Facilitating the sponsoring member and the member of the shared risk pool to access the probational member status may be providing access to the probational member status for the sponsoring member and the member of the shared risk pool. Access to the probational member status may be provided through an interface of a storage circuit, which may be accessed through a communication circuit.

In some examples, the method further comprises collecting a performance characteristic in real-time when the probational member is driving the first device through a telematics interface unit coupled to the first device for compiling the probational member status.

In some examples, the method further comprises collecting a performance characteristic in real-time for monitoring health of the probational member through a biometric monitor unit coupled to the probational member for compiling the probational member status.

In some examples, the method further comprises monitoring acceleration, braking, speed, swerving, use of lighting and turn signals of the first device by the probational member for compiling the probational member status.

In some examples, the method further comprises monitoring body temperature, heart rate, breathing rate, oxygen saturation, and regularity of the heartbeat in real-time of the probational member for compiling the probational member status.

In some examples, the method further comprises providing feedback to the sponsoring member for coaching the probational member.

In some examples, the method further comprises facilitating the member of the shared risk pool to vote on the probational member joining the shared risk pool.

An embodiment of the present invention provides a compute system, including a control unit configured to: analyze telematics data of a first device operated by a probational member; receive biometric data of the probational member; identify map data based on the telematics data, the biometric data, or a combination thereof; compile a probational member status based on the telematics data, biometric data, the map data, or a combination thereof concurrently compiled by real-time monitoring the probational member; facilitate a sponsoring member and a member of a shared risk pool to access the probational member status; and generate a determination of a vote by the sponsoring member and the member for an admission of the probational member to the shared risk pool based on the probational member status.

In some examples, the control unit is configured to collect a performance characteristic in real-time when the probational member is driving the first device through a telematics interface unit coupled to the first device for compiling the probational member status.

In some examples, the control unit is configured to collect a performance characteristic in real-time for monitoring health of the probational member through a biometric monitor unit coupled to the probational member for compiling the probational member status.

In some examples, the control unit is coupled to a telematics interface unit to monitor acceleration, braking, speed, swerving, use of lighting and turn signals of the first device by the probational member for compiling the probational member status.

In some examples, the control unit is coupled to a biometric monitor unit to monitor body temperature, heart rate, breathing rate, oxygen saturation, and regularity of the heartbeat in real-time of the probational member for compiling the probational member status.

In some examples, the system further comprises a storage circuit is coupled to a sponsor interface to provide feedback to the sponsoring member for coaching the probational member.

In some examples, the system further comprises a storage circuit configured to facilitate the member of the shared risk pool to vote on the probational member joining the shared risk pool.

An embodiment of the present invention provides a non-transitory computer readable medium including instructions for a compute system, including: analyzing telematics data of a first device operated by a probational member; receiving biometric data of the probational member; identifying map data based on the telematics data, the biometric data, or a combination thereof; compiling a probational member status based on the telematics data, biometric data, the map data, or a combination thereof concurrently compiled by real-time monitoring a the probational member; facilitating a sponsoring member and a member of a shared risk pool to access the probational member status; and generating a determination of a vote by the sponsoring member and the member for an admission of the probational member to the shared risk pool based on the probational member status.

Certain embodiments of the invention have other steps or elements in addition to or in place of those mentioned above. The steps or elements will become apparent to those skilled in the art from a reading of the following detailed description when taken with reference to the accompanying drawings.

### BRIEF DESCRIPTION OF THE DRAWINGS

FIG. 1 is a block diagram of a compute system with a risk management mechanism in an embodiment of the present invention.
FIG. 2 is an example of a top plan view illustration of a vehicle for the compute system.
FIG. 3 is shown an exemplary control flow diagram of a health risk management mechanism of the compute system.
FIG. 4 is shown an exemplary control flow diagram of an automobile risk management mechanism of the compute system.
FIG. 5 is an exemplary block diagram of the compute system in an embodiment.
FIG. 6 is a flow chart of a method of operation of a compute system in an embodiment of the present invention.

### BEST MODE FOR CARRYING OUT THE INVENTION

The features described, on an individual basis, can be executed in combination without altering the claimed invention. The description of the individual features is for clarity and understanding of the depth and breadth of the claimed invention, without limitation on the combination of those features. The concurrent or interleaved execution of any combination of the features is possible.

The following embodiments are described in sufficient detail to enable those skilled in the art to make and use the invention. It is to be understood that other embodiments would be evident based on the present disclosure, and that system, process, or mechanical changes may be made without departing from the scope of an embodiment of the present invention.

In the following description, numerous specific details are given to provide a thorough understanding of the invention. However, it will be apparent that the invention may be practiced without these specific details. In order to avoid obscuring an embodiment of the present invention, some well-known circuits, system configurations, and process steps are not disclosed in detail.

The drawings showing embodiments of the system are semi-diagrammatic, and not to scale and, particularly, some of the dimensions are for the clarity of presentation and are shown exaggerated in the drawing figures. Similarly, although the views in the drawings for ease of description generally show similar orientations, this depiction in the figures is arbitrary for the most part. Generally, the invention can be operated in any orientation. The embodiments of various components as a matter of descriptive convenience and are not intended to have any other significance or provide limitations for an embodiment of the present invention.

One skilled in the art would appreciate that the format with which navigation information is expressed is not critical to some embodiments of the invention. For example, in some embodiments, navigation information is presented in the format of (X, Y, Z); where X and Y and Z are three coordinates that define the geographic location, i.e., a position of a vehicle, an artifact, or a pixel in an optical data frame.

The term "module" referred to herein can be implemented as or include software running on specialized hardware, hardware, or a combination thereof in the present invention in accordance with the context in which the term is used. For example, the software can be machine code, firmware, embedded code, and application software. The software can also include a function, a call to a function, a code block, or a combination thereof.

Also, for example, the hardware can be gates, circuitry, processor, computer, integrated circuit, integrated circuit cores, memory devices, a pressure sensor, an inertial sensor, a microelectromechanical system (MEMS), passive devices, physical non-transitory memory medium including instructions for performing the software function, a portion therein, or a combination thereof to control one or more of the hardware units or circuits. Further, if a "unit" is written in the system claims section below, the "unit" is deemed to include hardware circuitry for the purposes and the scope of the system claims.

The units in the following description of the embodiments can be coupled or attached to one another as described or as shown. The coupling or attachment can be direct or indirect without or with intervening items between coupled or attached modules or units. The coupling or attachment can be by physical contact or by communication between modules or units, such as wireless communication.

The term "fuse" or "fusing" as used herein refer to combining, concatenating, merging, or integrating as appropriate for the usage of the term. In the example of a network, fusing the views of adjacent cameras can identify elements that are displayed in more than one view from adjacent cameras.

It is also understood that the nouns or elements in the embodiments can be described as a singular instance. It is understood that the usage of singular is not limited to singular but the singular usage can be applicable to multiple instances for any particular item referred to by the noun or element in the application. Numerous instances can be the same or similar or can be different.

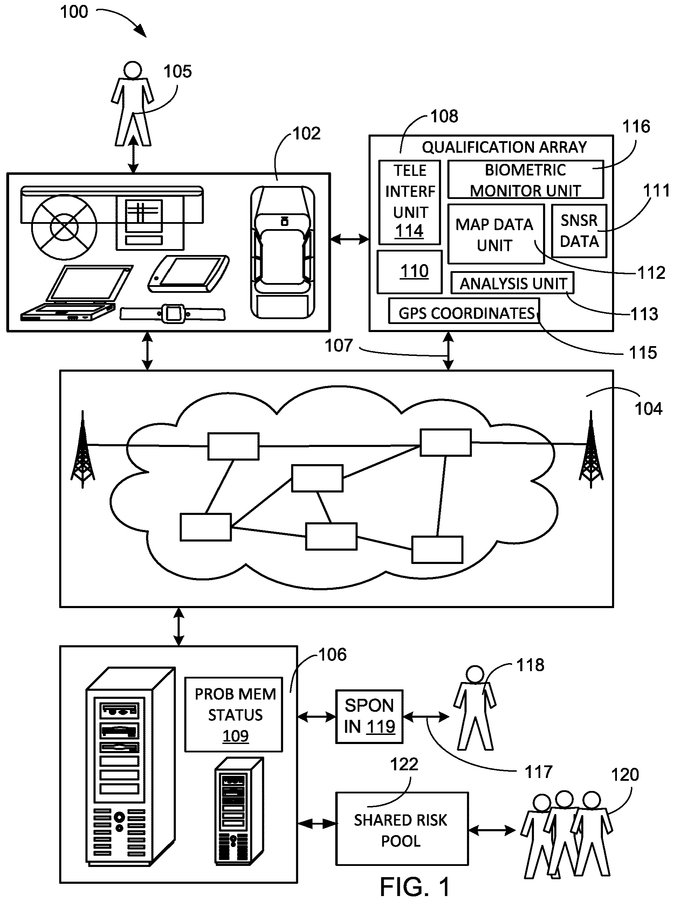

Referring now to FIG. 1, therein is shown a block diagram of a compute system 100 with a risk management mechanism in an embodiment of the present invention. The compute system 100 can include a first device 102, such as a client or a server, connected to a second device 106, such as a client or server.

The compute system 100 can include a system for capturing detailed information about a current environment based on fusion of multiple sources to reconcile and quickly identify specific details about the health or driving performance of a probational member 105. The first device 102 can communicate with the second device 106 through a network 104, such as a wireless or wired network. The probational member 105 can be an individual that has applied for a risk policy that can be linked to a shared risk pool of insured people, by nominating the probational member 105 to a sponsoring member 118 with a promise of a discount upon approval of the probational member 105.

For example, the first device 102 can be of any of a variety of computing devices, such as a cellular phone, personal digital assistant, a smart watch/health monitor, a notebook computer, an autonomous vehicle, automotive telematics navigation system, or other multifunctional device. Also, for example, the first device 102 can include a device or a subsystem, an autonomous or self-maneuvering vehicle or object, a driver assisted vehicle, a remote-controlled vehicle or object, or a combination thereof.

The first device 102 can couple, either directly or indirectly, to the network 104 to communicate with the second device 106 or can be a stand-alone device. The first device 102 can further be separate from or incorporated with a vehicle, such as a car, truck, bus, or motorcycle.

For illustrative purposes, the compute system 100 is described with the first device 102 as a mobile computing device, although it is understood that the first device 102 can be different types of devices. The first device 102 can be an autonomous vehicle or an accessory device coupled to the telematics vehicle network to support an autonomous vehicle.

The second device 106 can be any of a variety of centralized or decentralized computing devices. For example, the second device 106 can be a computer, grid computing resources, a virtualized computer resource, cloud computing resource, routers, switches, peer-to-peer distributed computing devices, or a combination thereof.

The second device 106 can be centralized in a single room, distributed across different rooms, distributed across different geographical locations, embedded within a telecommunications network. The second device 106 can couple with the network 104 to communicate with the first device 102. The second device 106 can also be a client type device as described for the first device 102.

For illustrative purposes, the compute system 100 is described with the second device 106 as a non-mobile computing device, although it is understood that the second device 106 can be different types of computing devices. For example, the second device 106 can also be a mobile computing device, such as notebook computer, another client device, a wearable device, or a different type of client device.

Also, for illustrative purposes, the compute system 100 is described with the second device 106 as a computing device, although it is understood that the second device 106 can be different types of devices. Also, for illustrative purposes, the compute system 100 is shown with the second device 106 and the first device 102 as endpoints of the network 104, although it is understood that the compute system 100 can include a different partition between the first device 102, the second device 106, and the network 104. For example, the first device 102, the second device 106, or a combination thereof can also function as part of the network 104.

The network 104 can span and represent a variety of networks. For example, the network 104 can include wireless communication, wired communication, optical, ultrasonic, or the combination thereof. Satellite communication, cellular communication, Bluetooth, Infrared Data Association standard (IrDA), wireless fidelity (WiFi), and worldwide interoperability for microwave access (WiMAX) are examples of wireless communication that can be included in the communication path. Ethernet, digital subscriber line (DSL), fiber to the home (FTTH), and plain old telephone service (POTS) are examples of wired communication that can be included in the network 104. Further, the network 104 can traverse a number of network topologies and distances. For example, the network 104 can include direct connection, personal area network (PAN), local area network (LAN), metropolitan area network (MAN), wide area network (WAN), or a combination thereof.

The compute system 100 can provide additional features that are not available in prior art compute systems. The first device 102 can be coupled to a qualification array 108 that include a sensor data local storage 110. The qualification array 108 includes monitoring devices, such as a camera positioned to observe the first device 102, configured to monitor, observe, record, or a combination thereof the surroundings of the first device 102. The sensor data local storage 110 provides a non-transitory storage medium including the instructions accessed by a control unit 113 to process a sensor data stream 111 captured by the qualification array 108. The control unit 113 can be a hardware structure, including a processor, a cloud server or, a convolutional neural network, executing specialized software that provides machine learning and artificial intelligence (AI) that can concurrently assemble data from the sensor data stream 111, telematics data from a telematics interface unit 114, biometrics data from a biometric monitor unit 116, map data from a map data unit 112 and GPS coordinates 115. The control unit 113 can compile a performance characteristic 107 of the probational member 105 in real-time for analysis. The performance characteristic 107 can include a real-time compilation of the driving statistics, the health statistics, or a combination thereof of based on the activities of the probational member 105.

By way of an example, the driving statistics can be developed from the concurrent analysis of the sensor data stream 111, the telematics data 114, map data 112, and the GPS coordinates 115 while the probational member 105 utilizes the first device 102. Also by way of an example, the health statistics can be concurrently compiled through the biometrics data 116 and the sensor data stream 111 in real-time analysis of the probational member 105 during normal activities. The control unit 113 can compile concurrent data from the map data unit 112, the telematics interface unit 114, and the biometric monitor unit 116 to generate the performance characteristic 107 of the probational member 105, The control unit 113 can compare the performance characteristic 107 to an accepted model learned during a training process in order to generate the performance characteristic 107.

The sensor data local storage 110 can be a hardware structure that includes the non-transitory storage medium for the specialized software including the instructions that implement the compute system 100. By way of an example, the sensor data local storage 110 can include volatile dynamic memory, non-volatile storage memory, such as Flash memory, optical media, or a combination thereof.

The map data unit 112 can be a hardware structure that provides navigation information and route data including speed limits, roadway status, and points of interest. The map data unit 112 can include circuitry that analyzes the actual route managed by the probational member 105 for evaluation by the control unit 113 based on feedback from the qualification array 108. The biometric monitor unit 116 can be a hardware device that collects physical data from the probational member 105 including body temperature, heart rate, breathing rate, oxygen saturation, and regularity of the heart beat for interpreting the physical condition of the probational member 105.

The sensor data stream 111 can fuse the information from the map data unit 112, the telematics interface unit 114, the GPS coordinates 115, and the biometric monitor unit 116. The sensor data stream 111 can be stored in the sensor data local storage 110 for processing by the control unit 113. The map data unit 112 can retrieve the local map information, based on the GPS coordinates 115, including speed limits, road types, current traffic conditions, and weather conditions from the second device 106 for monitoring the driving performance of the probational member 105. The control unit 113 can identify a current location by the GPS coordinates 115 and display the current location with information from the map data unit 112. The control unit 113 can determine the current speed and location of the vehicle 102 by monitoring the change in location over time. The control unit 113 can also retrieve telematics data from the telematics interface unit 114 to monitor the vehicle control performed by the probational member 105.

The telematics interface unit 114 can be a hardware interface unit configured to present status of the subsystems of the first device 102, such as a vehicle 102. The control unit 113 can monitor the driving performance of the probational member 105. The control unit 113 can compare the actual driving style of the probational member 105 to a safe driver model in order to present the performance characteristic 107 to the second device 106. The first device 102 can be a smart watch/health monitor 102, worn by the probational member 105, that can communicate through the sensor data stream 111 with the control unit 113 in order to track the physical condition of the probational member 105. The physical condition of the probational member 105 can be represented by respiration rate, heart rate, body temperature, oxygen saturation, regularity of the heart beat as analyzed by the biometric monitor unit 116 communicating to the control unit 113. The second device 106 can compile a probational member status 109 from the updates of the performance characteristic 107.

The map data unit 112 can be, a hardware structure capable of maintaining map and routing data, coupled to a telematics interface unit 114, and the sensor data local storage 110. The sensor data local storage 110 can be a hardware structure capable of receiving fixed size frames from each of the units of the qualification array 108, such as 200 to 300 times per second, periodically capturing the sensor data stream 111 that can be analyzed by the control unit 113 for determining the performance of the probational member 105. The telematics interface unit 114 can be a hardware structure that receives the sensor data stream 111. The telematics interface unit 114 also receives GPS coordinates 115 from the first device 102. Once processed, the sensor data stream 111 can represent the actual movement and performance of the probational member 105. It is understood that the GPS coordinates 115 can include latitude, longitude, and elevation of a single point or pixel in the sensor data stream 111.

For example, the sensor data local storage 110 can be implemented in a number of ways, such as a non-volatile storage device, such as a hard disk drive, a solid state storage device (SSD), a FLASH memory card, or a combination thereof capable of fusing the sensor data stream 111 from each of the elements of the qualification array 108 for further processing by the control unit 113.

The sensor data local storage 110 can store the sensor data stream 111, including sampled frames of data from each of the units in the qualification array 108, in order to provide the control unit 113 with relative locations of features based on the physical location of the first device 102. The control unit 113 can evaluate the driving performance of the probational member 105 in order to compile the performance characteristic 107. The performance characteristic 107 can be an analysis of the performance of the probational member 105 as compared to a safe driver profile by the control unit 113.

The probational member status 109 can be compiled by the second device 106 to provide a sponsor interface 119 with feedback 117 presented to a sponsoring member 118 for coaching the probational member 105. The feedback 117 can include a health condition, driving proficiency, or a combination thereof. The sponsoring member 118 can have a relationship or knowledge of the probational member 105. By proposing the candidacy of the probational member 105, the sponsoring member 118 and receive a discount in the cost of their vehicle or health risk management policy. The probational member 105 can be influenced by peer pressure to improve their driving performance in order to benefit the sponsoring member 118. The probational member 105 can be evaluated for a qualification period before being voted on by members 120 of a shared risk pool 122. The members 120 of the shared risk pool 122 can be the members 120 of a shared risk pool of insured individuals that can share in a reduced cost of their risk management costs as a "family" group or commercial group of risk pool for the risk management company.

By way of an example, the probational member 105 can be a relative or acquaintance of the sponsoring member 118. After the qualification period, the sponsoring member 118 can make a recommendation to the members 120 of the shared risk pool 122 to accept or reject the probational member 105 as a member of the members 120 of the shared risk pool 122. The members 120 of the shared risk pool 122 can vote for an admission of the probational member 105. The voting process can be unanimous, a majority, or a percentage threshold as agreed by the risk management company and the members 120 of the shared risk pool 122. It is understood that the sponsoring member 118 can be any one of the members 120 of the shared risk pool 122.

By way of an example, the sponsoring member 118 can receive a 5% discount in the risk management cost when the probational member 105 is accepted into the members 120 of the shared risk pool 122. Each of the members 120 of the shared risk pool 122 can receive a 1% reduction in costs of their risk management policy by accepting the probational member 105. The members 120 of the shared risk pool 122 can motivate each other to maintain healthy habits as well as good driving habits in order to keep good standing in the members 120 of the shared risk pool 122. This process can reduce the overall costs for the risk management company, while saving money for the members 120 of the shared risk pool 122.

It is understood that a risk management company can reduce their risk with a larger pool of people in the shared risk pool 122 of people. The risk management policy costs can be distributed across the entire shared risk pool 122. By increasing the count of the members 120 of the shared risk pool 122, the overall cost of the risk management policy can be reduced. The members 120 of the shared risk pool 122 can represent a risk management "family" of elected individuals.

It has been discovered that the compute system 100 with a risk management mechanism can improve the process for insuring individuals by allowing the probational member 105 to be evaluated over time in order to qualify to join the members 120 of the shared risk pool 122. The compute system 100 can reduce risk for the risk management company by applying peer pressure to the probational member 105 in order to improve their health and driving abilities over the qualification period. By identifying a sponsoring member 118, the probational member 105 is incentivized to improve their health, driving capabilities, or a combination thereof in order to respect the sponsoring member 118. The compute system 100 can improve the risk evaluation process over the existing statistical method by providing the probational member status 109 detailing the exact characteristics of the probational member 105 as opposed to a general statistic of a broad population. This can improve the risk management registration process and reduce costs for the risk management company and the members 120 of the shared risk pool 122.

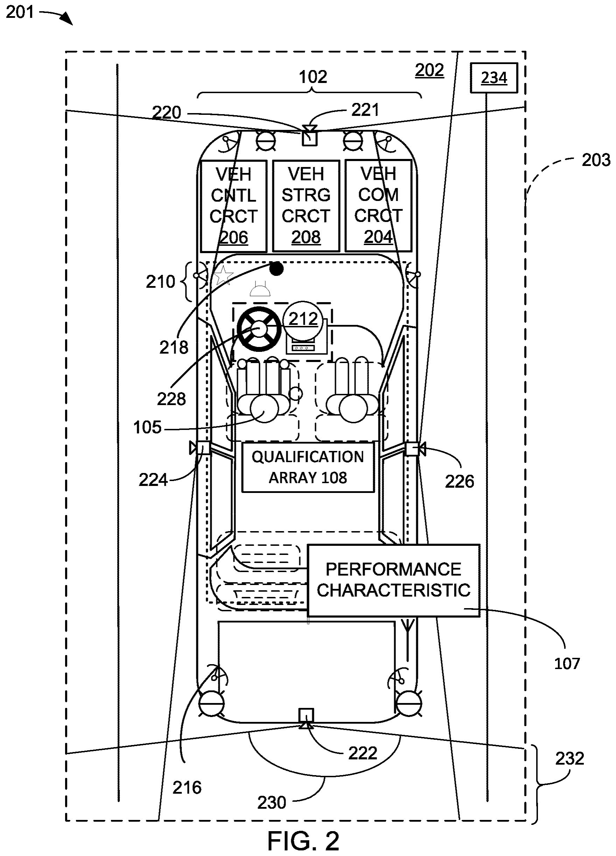

Referring now to FIG. 2, therein is shown an example of a top plan view 201 illustration of the vehicle 102 for the compute system 100 of FIG. 1. The compute system 100 can be included in or interact with the first device 102. The probational member 105 can authorize the use of the qualification array 108 of FIG. 1 to monitor the health and driving capabilities of the probational member 105 during the qualification period.

The first device 102 can be an object or a machine used for transporting people or goods capable of automatically maneuvering or operating the object or the machine. The first device 102 can include vehicles accessible by the probational member 105 for control, maneuver, operation, or a combination thereof. For example, the first device 102 can include a car, a truck, a cart, a drone, or a combination thereof. The presence of the qualification array 108 can monitor the health and driving performance of the probational member 105 during the qualification period.

The first device 102 can include a front optical sensor 220, a rear optical sensor 222, a left optical sensor 224, and a right optical sensor 226 for monitoring the relative position of the first device 102. Each of the front optical sensor 220, the rear optical sensor 222, the left optical sensor 224, and the right optical sensor 226 can be a hardware camera including a fisheye lens 221 having a 170 degree view of a region of interest 203.

The first device 102 or other vehicles interfacing with the compute system 100 can include a device, a circuit, one or more specific sensors, such as environmental sensors 210, or a combination thereof for providing assistance or additional information to the sensor data stream 111 to monitor the controlling, maneuvering, or operating the first device 102 by the probational member 105. The environmental sensors 210 can include a cabin camera, LiDAR sensors, the front optical sensor 220, the rear optical sensor 222, the left optical sensor 224, and the right optical sensor 226, or a combination thereof. The telematics interface unit 114 of FIG. 1 can interface with a camera with a wide-angle lens, such as the fisheye lens 221. The qualification array 108 can be mounted on the interior of the first device 102 positioned at the front, rear, left side, and right side of the first device 102. The first device 102 or any other vehicles can include a vehicle communication circuit 204, a vehicle control circuit 206, a vehicle storage circuit 208, other interfaces, or a combination thereof.

The vehicle storage circuit 208 can include a functional unit or circuit integral to the corresponding first device 102 and configured to store and recall information. The vehicle storage circuit 208 can be a volatile memory, a nonvolatile memory, an internal memory, an external memory, or a combination thereof and can interface with the sensor data local storage 110 of FIG. 1. For example, the vehicle storage circuit 208 can be a nonvolatile storage such as non-volatile random access memory (NVRAM), Flash memory, disk storage, or a volatile storage such as static random access memory (SRAM).

The vehicle storage circuit 208 can store vehicle software, other relevant data, such as input information, information from sensors, processing results, information predetermined or preloaded by the compute system 100 or vehicle manufacturer, or a combination thereof.

The vehicle control circuit 206 can include a functional unit or circuit integral to the first device 102 and configured as a processor to execute or implement instructions. The vehicle control circuit 206 can execute or implement the vehicle software to provide the intelligence of the corresponding vehicle, the compute system 100, or a combination thereof.

The vehicle control circuit 206 can be implemented in a number of different manners. For example, the vehicle control circuit 206 can be a processor, an application specific integrated circuit (ASIC) an embedded processor, a microprocessor, a hardware control logic, a hardware finite state machine (FSM), a digital signal processor (DSP), or a combination thereof. As a more specific example, the vehicle control circuit 206 can include an engine control unit, one or more central processing unit, or a combination thereof.

The vehicle communication circuit 204 can include a functional unit or circuit integral to the corresponding vehicle, such as the first device 102. The vehicle communication circuit 204 can be configured to enable external communication to and from the corresponding vehicle. For example, the vehicle communication circuit 204 can permit the first device 102 to communicate with the second device 106 through the network 104 of FIG. 1 including transferring the performance characteristic 107.

The vehicle communication circuit 204 can also function as a communication hub allowing the corresponding vehicle to function as part of the network 104 and not limited to be an end point or terminal circuit to the network 104. The vehicle communication circuit 204 can include active and passive components, such as microelectronics, circuitry, or an antenna, for interaction with the network 104. For example, the vehicle communication circuit 204 can include a modem, a transmitter, a receiver, a port, a connector, or a combination thereof for wired communication, wireless communication, or a combination thereof.

The vehicle communication circuit 204 can couple with the network 104 to send or receive information directly between the vehicle communication circuit 204 and the second device 106 as end points of the communication, such as for direct line-of-sight communication or peer-to-peer communication. The vehicle communication circuit 204 can further couple with the network 104 to send or receive information through a server or another intermediate device in between endpoints of the communication.

The first device 102 or other vehicles can further include various interfaces. The first device 102 can include one or more interfaces for interaction or internal communication between functional units or circuits of the first device 102. For example, the first device 102 can include one or more interfaces, such as drivers, firmware, wire connections or buses, protocols, or a combination thereof, for the vehicle storage circuit 208, the vehicle control circuit 206, or a combination thereof. By way of an example, the vehicle communication circuit 204 can communicate with the telematics interface unit 114 of FIG. 1 of the qualification array 108 to report on the control of the first device 102.

The first device 102 can further include one or more interfaces for interaction with an occupant, an operator or a passenger, the probational member 105, or a combination thereof. For example, the first device 102 can include a user interface 212 including input or output devices or circuits, such as a screen or touch screen, a speaker, a microphone, a keyboard or other input devices, an instrument panel, or a combination thereof.

The first device 102 can further include one or more interfaces along with switches or actuators for physically controlling movable components of the first device 102. For example, the first device 102 can include the one or more interfaces along with the monitoring mechanisms to log the physical performance and control of the maneuvering of the first device 102.

The functional units or circuits in the first device 102 can work individually and independently of the other functional units or circuits. The first device 102 can work individually and independently from the network 104, the second device 106, other devices or vehicles, or a combination thereof.

The functional units or circuits described above can be implemented in hardware. For example, one or more of the functional units or circuits can be implemented using a gate, circuitry, a processor, a computer, integrated circuit, integrated circuit cores, a pressure sensor, an inertial sensor, a microelectromechanical system (MEMS), a passive device, a physical non-transitory memory medium containing instructions for performing the software function, a portion therein, or a combination thereof.

The environmental sensors 210 are each a device or a circuit for detecting or identifying environment of the corresponding vehicle. The environmental sensors 210 can detect, identify, determine, or a combination thereof, such as for status, surroundings or movement for the corresponding vehicle. The environmental sensors 210 can detect, identify, determine, or a combination thereof for environment within a cabin of the corresponding vehicle, an environment external to and surrounding the corresponding vehicle, or a combination thereof. The environmental sensors 210 can be implement for the first device 102.

For example, the environmental sensors 210 can include a user interface 212, a radar sensor 216, a location-movement sensor 218, the front optical sensor 220, the rear optical sensor 222, the left optical sensor 224, and the right optical sensor 226, or a combination thereof. The user interface 212 can include a projector, a video screen, a touch screen, a speaker, or any combination thereof. The user interface 212 can display a planned route, lane suggestions, speed warnings, vehicle system alerts and combinations thereof.

The radar sensor 216 can include an object-detection system, device, or circuit. The radar sensor 216 can determine or identify an existence of an object or a target, such as an obstacle or another vehicle, external to the corresponding device or vehicle, a relative location or a distance between the object or the target and the corresponding device or vehicle, or a combination thereof.

The radar sensor 216 can utilize radio waves to determine or identify an existence of the object or the target, the relative location or a distance relative to the first device 102 or other corresponding device or vehicle, or a combination thereof. For example, the radar sensor 216 can include a proximity sensor or warning system, such as for an area in front of, behind, adjacent to or on a side of, or a combination thereof geographically or physically relative to the first device 102.

The location-movement sensor 218 can be a sensor for identifying or calculating a geographic location of the corresponding vehicle or device, determining a movement or speed of the corresponding vehicle or device, or a combination thereof. The location-movement sensor 218 can include an accelerometer, a speedometer, a Global Positioning System (GPS) receiver or device, a gyroscope or a compass, or a combination thereof. The first device 102 can include the environmental sensors 210 other than or in addition to the location-movement sensor 218. The location-movement sensor 218 can provide a gyroscope rate of change for monitoring turns and a speed from the speedometer.

The compute system 100 can use the qualification array 108 corresponding to one or more devices, one or more vehicles, or a combination thereof to generate the performance characteristic 107 describing or representing information regarding the environment surrounding the corresponding device or vehicle. The sensor data stream 111 of FIG. 1 can be further processed with the vehicle control circuit 206, stored in the vehicle storage circuit 208, communicated to another device or vehicle through the vehicle communication circuit 204, or a combination thereof.

As a more specific example, the vehicle communication circuit 204, the vehicle control circuit 206, the vehicle storage circuit 208, the qualification array 108, one or more interfaces, or a combination thereof can be included in or make up the first device 102.

The compute system 100 can provide a vehicle movement control 228 as a suggestion to the sponsoring member 118 for maneuvering or operating the first device 102. Details regarding the utilization and processing of the vehicle movement control 228 are discussed below.

The compute system 100 can process and monitor the vehicle movement control 228 for maneuvering the first device 102. The vehicle movement control 228 is an instruction, a signal, a process, a method, a mechanism, or a combination thereof for monitoring the physical movement or travel of the first device 102.

Continuing with the example, the compute system 100 can use the vehicle movement control 228 provided from the first device 102 as an input to the performance characteristic 107. The compute system 100 can utilize the vehicle movement control 228 to provide information, on the control of the first device 102.

Continuing with the example, the compute system 100 can communicate the performance characteristic 107 through the second device 106 to other devices or vehicles, or directly communicate to the other devices or vehicles, such as for a peer-to-peer communication system. The compute system 100 can generate the probational member status 109 in the second device 106 by compiling updates of the performance characteristic 107 for informing other devices or vehicles of the status of the first device 102 itself, about other vehicles detected and identified around the first device 102, or a combination thereof.

As a more specific example, the compute system 100 can use the probational member status 109 to detect weaknesses in the safe operation of the first device 102, as detected by the vehicle movement control 228, such as for steering, braking, setting or adjusting travel speed, accessary control, or a combination thereof. Details regarding the processing of the vehicle movement control 228 are discussed below.

It is understood that the qualification array 108 can be positioned at fixed locations around the first device 102. By way of an example, the front optical sensor 220, the rear optical sensor 222, the left side optical sensor 224, and the right optical sensor 226 can monitor the region of interest 203 including the details of the roadway 202. The combination of the sensor data streams 111 of FIG. 1 from each of the front optical sensor 220, the rear optical sensor 222, the left side optical sensor 224, and the right optical sensor 226 can be fused by the map data unit 112 of FIG. 1 to form surrounding view of the region of interest 203.

It is understood that due to the characteristics of the wide-angle lenses and the relative position of the first device 102, objects can appear in more than one of the optical data streams 111. By way of an example, a fisheye viewing angle 230 can generate overlap regions 232 at the four corners of the region of interest 203. It is understood that the fisheye viewing angle 230 can be substantially equal to 170 degrees of view. The resulting ones of the overlap region 232 can be resolved by the vehicle control circuit 206. By way of an example, a traffic control sign 234 can be viewed by both the front optical sensor 220 and the right optical sensor 226. The vehicle control circuit 206 can resolve the traffic control signal 234 to be a single unit of the traffic control sign 234 adjacent to the roadway 202.

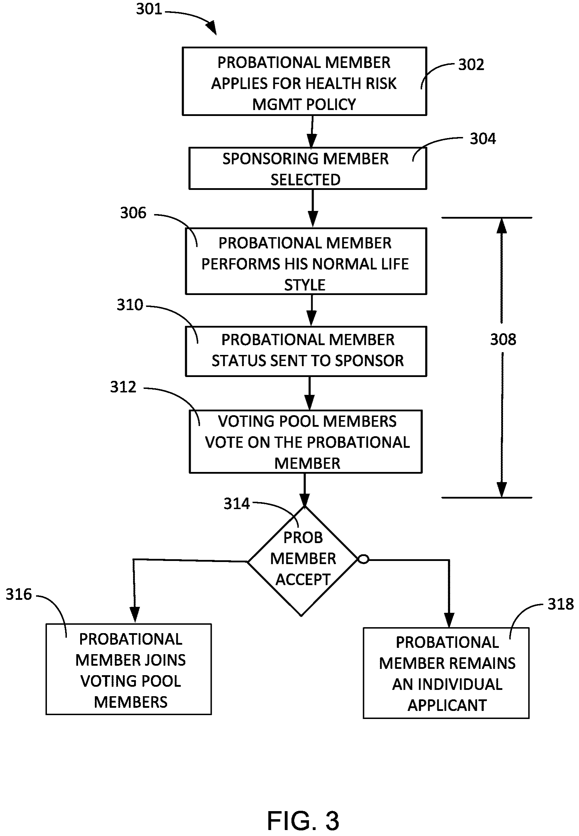

Referring now to FIG. 3, therein is shown an exemplary control flow diagram of a health risk management mechanism 301 of the compute system 100. The health risk management mechanism 301 depicts a probational member applies for health risk management policy block 302, which includes the probational member 105 applying for a health risk management policy that can be eligible to join the shared risk pool 122 of FIG. 1. The probational member 105 must agree to being monitored by the qualification array 108 and share the health information with a sponsoring member 118. The first device 102 can be a health monitoring smart watch 102 that communicates with the qualification array 108 through the biometric monitor unit 116 of FIG. 1 in order to generate the performance characteristic 107 of FIG. 1.

The health risk management mechanism 301 proceeds to a sponsoring member selected block 304, in which the sponsoring member 118 can be selected by the risk management company and agreed to by the probational member 105. The sponsoring member 118 can be motivated to accept the responsibility by the offer of a discount in their own risk management premiums. In some embodiments, the sponsoring member 118 can promote the probational member 105 to the risk management company for a risk management premium reward. The sponsoring member 118 can be related to the probational member 105 or they can be nominated by the risk management company.

The health risk management mechanism 301 proceeds to a probational member performs his normal life style block 306, initiating a qualification period 308. The qualification period 308 can be defined by the risk management company based on the age and gender of the probational member 105. The qualification period 308 can be three months, six months, or a duration agreed upon by the probational member 105. During the qualification period 308, the probational member can be charged an initial amount for health coverage without a discount. The sponsoring member 118 can help guide the probational member 105 to improve their life style. The sponsoring member 118 can suggest the probational member 105 should stop smoking, lose some weight, stop using alcohol or drugs, and get more exercise.

The health risk management mechanism 301 proceeds to a probational member status sent to sponsoring member block 310, in which the sponsoring member 118 receives updates to the probational member status 109 of FIG. 1. The probational member 105 can wear a health monitor smart watch 102 that communicates with the qualification array 108 to monitor heart rate, breathing rate, body temperature, blood sugar, oxygen saturation, and regularity of the heart beat for interpreting the physical condition of the probational member 105. The sponsoring member 118 can receive updates of the probational member status 109 on a set frequency, such as hourly, daily, or weekly. The sponsoring member 118 can assist the probational member 105 to improve their physical condition and maintain a healthy life style.

At the end of the qualification period 308, the health risk management mechanism 301 proceeds to a members of the shared risk pool vote on the probational member block 312. The members 120 of FIG. 1 of the shared risk pool 122 of FIG. 1 can review the probational member status 109 and question the sponsoring member 118 prior to taking a vote on whether to accept the probational member 105 into the shared risk pool 122 as one of the members 120 of the shared risk pool 122.

The health risk management mechanism 301 proceeds to a probational member accept decision block 314. If the probational member 105 is accepted, the health risk management mechanism 301 proceeds to a probational member joins members of the shared risk pool block 316, in which the probational member 105 is allowed to join the members 120 of the shared risk pool 122 and all of the members 120 are rewarded with a discount, such as 1-2%, on the cost of their health risk management policy that they purchased and the sponsoring member is also rewarded with a discount, such as 3-5% on the cost of their health risk management policy. If the probational member 105 is not accepted, the health risk management mechanism 301 proceeds to a probational member remains an individual applicant block 318. The probational member remains an individual applicant block 318 terminates the probational member's attempt to join the shared risk pool and provides no discount on the cost of the health risk management policy. The sponsoring member 118 can be awarded a smaller discount, such as 1%, for helping the probational member 105 through the process.

It has been discovered that the health risk management mechanism 301 can reduce the risk associated with providing a health risk management policy to the probational member 105, while incentivizing the sponsoring member 118 to assist the probational member 105 to improve their life style and maintain a healthy conditioning. It is understood that the maintenance of a healthy life style by the probational member 105 can reduce the probability of health related issues and therefore reduce risk management company costs. By way of an example, the risk management company can be an insurance company that offers health insurance to individuals and groups.

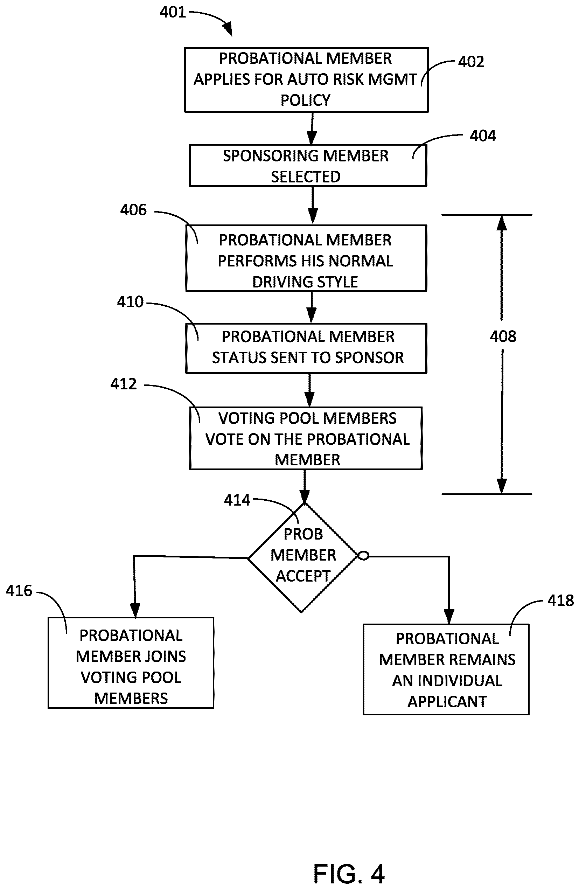

Referring now to FIG. 4, therein is an exemplary control flow diagram of an automobile risk management mechanism 401 of the compute system 100 of FIG. 1. The automobile risk management mechanism 401 depicts a probational member applies for an automobile risk management block 402, which includes the probational member 105 applying for an automobile risk management policy that can be eligible to join a shared risk pool 122 of the risk management company. The probational member 105 must agree to being monitored by the qualification array 108 and share the driving performance information with a sponsoring member 118. The first device 102 can be a vehicle 102 that communicates with the qualification array 108 through the telematics interface unit 114 of FIG. 1 in order to generate the performance characteristic 107 of FIG. 1.

The automobile risk management mechanism 401 proceeds to a sponsoring member selected block 404, in which the sponsoring member 118 can be selected by the risk management company and agreed to by the probational member 105. The sponsoring member 118 can be motivated to accept the responsibility by the offer of a discount in their own risk management policy premiums. In some embodiments, the sponsoring member 118 can promote the probational member 105 to the risk management company for a risk management premium reward. The sponsoring member 118 can be related to the probational member 105 or they can be nominated by the risk management company.

The automobile risk management mechanism 401 proceeds to a probational member performs his normal life style block 406, initiating a qualification period 408. The qualification period 408 can be defined by the risk management company based on the age and gender of the probational member 105. The qualification period 408 can be three months, six months, or a duration agreed upon by the probational member 105. During the qualification period 408, the probational member 105 can be charged an initial amount for automobile coverage without a discount. The sponsoring member 118 can help guide the probational member 105 to improve their driving style. The sponsoring member 118 can suggest the probational member 105 should use turn signals, obey speed limits, use caution changing lanes, use headlights at dusk and in the rain in order to improve the driving safety.

The automobile risk management mechanism 401 proceeds to a probational member status sent to sponsoring member block 410, in which the sponsoring member 118 receives updates to the probational member status 109 of FIG. 1. The probational member 105 can drive the vehicle 102 that communicates with the qualification array 108 through the telematics interface unit 114 to monitor acceleration, braking, swerving, use of lighting and turn signals by the probational member 105. The sponsoring member 118 can receive updates of the probational member status 109 on a set frequency, such as daily or weekly. The sponsoring member 118 can assist the probational member 105 to improve their driving style by not texting while driving, not exceeding the speed limit, braking more smoothly, not swerving through traffic, using turn signals when turning or changing lanes, and using headlights at dusk or in the rain in order to improve the driving style.

At the end of the qualification period 408, the automobile risk management mechanism 401 proceeds to a members of the shared risk pool vote on an admission of the probational member block 412. The members 120 of FIG. 1 of the shared risk pool 122 of FIG. 1 can review the probational member status 109 and question the sponsoring member 118 prior to taking a vote on whether to accept the probational member 105 into the shared risk pool 122 as one of the members 120 of the shared risk pool 122.

The automobile risk management mechanism 401 proceeds to a probational member accept decision block 414. If the probational member 105 is accepted, the automobile risk management mechanism 401 proceeds to a probational member joins members of the shared risk pool block 416, in which the probational member 105 is allowed to join the members 120 of the shared risk pool 122 and all of the members 120 can be rewarded with a discount, such as 1-2%, on the cost of their automobile risk management policy that they purchased and the sponsoring member is also rewarded with a discount, such as 3-5% on the cost of their automobile risk management policy. If the probational member 105 is not accepted, the automobile risk management mechanism 401 proceeds to a probational member remains an individual applicant block 418. The probational member remains an individual applicant block 418 terminates the probational member's attempt to join the shared risk pool and provides no discount on the cost of the health risk management policy. The sponsoring member 118 can be awarded a smaller discount, such as 1%, for helping the probational member 105 through the process.

It has been discovered that the automobile risk management mechanism 401 can reduce the risk associated with providing an automobile risk management policy to the probational member 105, while incentivizing the sponsoring member 118 to assist the probational member 105 to improve their driving style and maintain a good driving style. The automobile risk management mechanism 401 can improve the qualification and rate scaling process by evaluating the probational member 105 in actual operation of the first device 102 as opposed to relying on generalized statistics that can be incorrect. It is understood that the maintenance of the good driving style by the probational member 105 can reduce the probability of accident related issues and therefore reduce risk management company costs. By way of an example, the risk management company can be an insurance company that offers automobile insurance to individuals and groups.

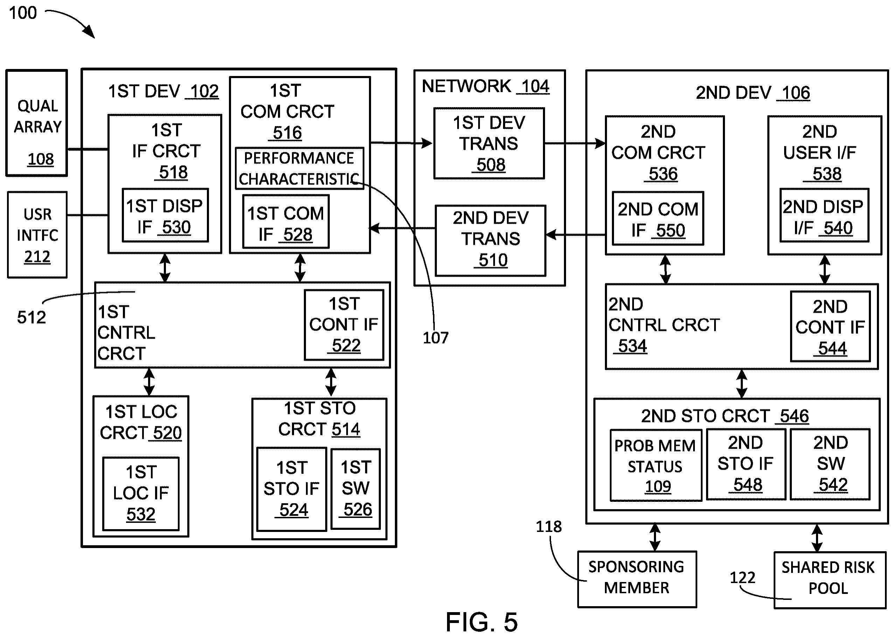

Referring now to FIG. 5, therein is shown an exemplary block diagram of the compute system 100 in an embodiment. The compute system 100 can include the first device 102, the network 104, and the second device 106. The first device 102 can send information in a first device transmission 508 over the network 104 to the second device 106. The second device 106 can send information in a second device transmission 510 over the network 104 to the first device 102.

For illustrative purposes, the compute system 100 is shown with the first device 102 as a client device, although it is understood that the compute system 100 can include the first device 102 as a different type of device. For example, the first device 102 can be a server containing the first display interface 530 coupled to the user interface 212.

Also, for illustrative purposes, the compute system 100 is shown with the second device 106 as a server, although it is understood that the compute system 100 can include the second device 106 as a different type of device. For example, the second device 106 can be a client device. By way of an example, the compute system 100 can be implemented entirely on the first device 102. The second device 106 can provide additional computing speed and power.

Also, for illustrative purposes, the compute system 100 is shown with interaction between the first device 102 and the second device 106. However, it is understood that the first device 102 can be a part of or the entirety of a vehicle, a smart vehicle, or a combination thereof. Similarly, the second device 106 can similarly interact with the first device 102 representing the autonomous vehicle, the intelligent vehicle, or a combination thereof.

For brevity of description in this embodiment of the present invention, the first device 102 will be described as a client device and the second device 106 will be described as a server device. The embodiment of the present invention is not limited to this selection for the type of devices. The selection is an example of an embodiment of the present invention.

The first device 102 can include a first control circuit 512, a first storage circuit 514, a first communication circuit 516, a first interface circuit 518, and a first location circuit 520. The first control circuit 512 can include a first control interface 522. The first control circuit 512 can execute a first software 526 to provide the intelligence of the compute system 100.

The first control circuit 512 can be implemented in a number of different manners. For example, the first control circuit 512 can be a processor, an application specific integrated circuit (ASIC) an embedded processor, a microprocessor, a hardware control logic, a hardware finite state machine (FSM), a digital signal processor (DSP), or a combination thereof. The first control interface 522 can be used for communication between the first control circuit 512 and other functional units or circuits in the first device 102. The first control interface 522 can also be used for communication that is external to the first device 102.

The first control interface 522 can receive information from the other functional units/circuits or from external sources, or can transmit information to the other functional units/circuits or to external destinations. The external sources and the external destinations refer to sources and destinations external to the first device 102.

The first control interface 522 can be implemented in different ways and can include different implementations depending on which functional units/circuits or external units/circuits are being interfaced with the first control interface 522. For example, the first control interface 522 can be implemented with a pressure sensor, an inertial sensor, a microelectromechanical system (MEMS), optical circuitry, waveguides, wireless circuitry, wireline circuitry, analogue circuitry, or a combination thereof.

The first storage circuit 514 can store the first software 526. The first storage circuit 514 can also store the relevant information, such as data representing incoming images, data representing previously presented image, sound files, or a combination thereof.

The first storage circuit 514 can be a volatile memory, a nonvolatile memory, an internal memory, an external memory, or a combination thereof. For example, the first storage circuit 514 can be a nonvolatile storage such as non-volatile random-access memory (NVRAM), Flash memory, disk storage, or a volatile storage such as static random-access memory (SRAM).

The first storage circuit 514 can include a first storage interface 524. The first storage interface 524 can be used for communication between the first storage circuit 514 and other functional units or circuits in the first device 102, such as the sensor data local storage 110 of FIG. 1. The first storage interface 524 can also be used for communication that is external to the first device 102.

The first storage interface 524 can be a hardware circuitry configured to receive information from the other functional units/circuits or from external sources, or can transmit information to the other functional units/circuits or to external destinations. The external sources and the external destinations refer to sources and destinations external to the first device 102.

The first storage interface 524 can include different implementations depending on which functional units/circuits or external units/circuits are being interfaced with the first storage circuit 514. The first storage interface 524 can be implemented with technologies and techniques similar to the implementation of the first control interface 522, such as analogue circuitry, digital circuitry, wireless circuitry, or wireline circuitry.

The first communication circuit 516 can enable external communication to and from the first device 102. For example, the first communication circuit 516 can permit the first device 102 to communicate with the second device 106 and the network 104.

The first communication circuit 516 can also function as a communication hub allowing the first device 102 to function as part of the network 104 and not limited to be an endpoint or terminal circuit to the network 104. The first communication circuit 516 can include active and passive components, such as microelectronics, analogue circuitry, wireless circuitry, wireline circuitry, or an antenna, for interaction with the network 104.

The first communication circuit 516 can include a first communication interface 528. The first communication interface 528 can be used for communication between the first communication circuit 516 and other functional units or circuits in the first device 102. The first communication interface 528 can receive information from the second device 106 for distribution to the other functional units/circuits or can transmit information to the other functional units or circuits.

The first communication interface 528 can include different implementations depending on which functional units or circuits are being interfaced with the first communication circuit 516. The first communication interface 528 can be implemented with technologies and techniques similar to the implementation of the first control interface 522, including a microelectromechanical system (MEMS), optical circuitry, waveguides, wireless circuitry, wireline circuitry, analogue circuitry, or a combination thereof.

The first interface circuit 518 allows the qualification array 108 to interface and interact with the first device 102. The control unit 113 of FIG. 1, of the qualification array 108, can communicate through the first device 102. The first interface circuit 518 can include an input device and an output device. Examples of the input device of the first interface circuit 518 can include a keypad, a touchpad, soft-keys, a keyboard, a microphone, a radio frequency receiver, an infrared sensor for receiving remote signals, the optical sensor 108, or any combination thereof to provide data and communication inputs. By way of an example, the optical sensor 108 can connect to the first interface circuit 518 through a wired or wireless connection. The first interface circuit 518 can pass the input from the optical sensor 108 to the first control circuit 512 for processing and storage.

The first interface circuit 518 can include a first display interface 530. The first display interface 530 can include an output device. The first display interface 530 can couple the user interface 212 including a projector, a video screen, a touch screen, a speaker, a microphone, a keyboard, and combinations thereof.

The first control circuit 512 can also execute the first software 526 for the other functions of the compute system 100, including receiving location information from the first location circuit 520. The first control circuit 512 can further execute the first software 526 for interaction with the network 104 via the first communication circuit 516.

The first location circuit 520 can be a hardware circuit configured to generate location information used to identify real world coordinates, such as the GPS coordinates 115 of FIG. 1. The first control circuit 512 can calculate the geolocation of the first device 102 as determined by the location circuit 520 including the geolocation of the qualification array 108. The first location circuit 520 can be implemented in many ways. For example, the first location circuit 520 can function as at least a part of the global positioning system, an inertial compute system, a cellular-tower location system, a gyroscope, or any combination thereof. Also, for example, the first location circuit 520 can utilize components such as an accelerometer, gyroscope, or global positioning system (GPS) receiver in order to identify the current location on the Earth by satellite triangulation of cell tower triangulation, including calculating pseudo ranges to the satellites or the cell towers.

The first location circuit 520 can include a first location interface 532. The first location interface 532 can be used for communication between the first location circuit 520 and other functional units or circuits in the first device 102, including the optical sensor 108.

The first location interface 532 can receive information from the other functional units/circuits or from external sources, or can transmit information to the other functional units/circuits or to external destinations. The external sources and the external destinations refer to sources and destinations external to the first device 102, including satellites and cell towers.

The first location interface 532 can include different implementations depending on which functional units/circuits or external units/circuits are being interfaced with the first location circuit 520. The first location interface 532 can be implemented with technologies and techniques similar to the implementation of the first control circuit 512, including analogue circuitry, digital circuitry, wireless circuitry, or wireline circuitry.

The second device 106 can be optimized for implementing an embodiment of the present invention in a multiple device embodiment with the first device 102. The second device 106 can provide the additional or higher performance processing power compared to the first device 102. The second device 106 can include a second control circuit 534, a second communication circuit 536, a second user interface 538, and a second storage circuit 546.

The second user interface 538 allows an operator (not shown) to interface and interact with the second device 106. The control unit 113, of the qualification array 108, can communicate directly to the second user interface 538 as an option. The second user interface 538 can include an input device and an output device. Examples of the input device of the second user interface 538 can include a keypad, a touchpad, soft-keys, a keyboard, a radio frequency receiver, a microphone, or any combination thereof to provide data and communication inputs. Examples of the output device of the second user interface 538 can include a second display interface 540. The second display interface 540 can include a display, a projector, a video screen, a speaker, or any combination thereof.

The second control circuit 534 can execute a second software 542 to provide the intelligence of the second device 106 of the compute system 100. The second software 542 can operate in conjunction with the first software 526. The second control circuit 534 can provide additional performance compared to the first control circuit 512.

The second control circuit 534 can operate the second user interface 538 to display information. The second control circuit 534 can also execute the second software 542 for the other functions of the compute system 100, including operating the second communication circuit 536 to communicate with the first device 102 over the network 104.

The second control circuit 534 can be implemented in a number of different manners. For example, the second control circuit 534 can be a processor, an embedded processor, a microprocessor, hardware control logic, a hardware finite state machine (FSM), a digital signal processor (DSP), or a combination thereof.

The second control circuit 534 can include a second control interface 544. The second control interface 544 can be used for communication between the second control circuit 534 and other functional units or circuits in the second device 106. The second control interface 544 can also be used for communication that is external to the second device 106. The second control circuit 534 can receive updates of the performance characteristic 107 to compile the probational member status 109.

The second control interface 544 can receive information from the other functional units/circuits or from external sources, or can transmit information to the other functional units/circuits or to external destinations. The external sources and the external destinations refer to sources and destinations external to the second device 106.

The second control interface 544 can be implemented in different ways and can include different implementations depending on which functional units/circuits or external units/circuits are being interfaced with the second control interface 544. For example, the second control interface 544 can be implemented with a pressure sensor, an inertial sensor, a microelectromechanical system (MEMS), optical circuitry, waveguides, wireless circuitry, wireline circuitry, analogue circuitry, or a combination thereof.

The second storage circuit 546 can store the second software 542. The second storage circuit 546 can also store the information such as the probational members status 109 captured by the qualification array 108. The second storage circuit 546 can be sized to provide the additional storage capacity to supplement the first storage circuit 514.

For illustrative purposes, the second storage circuit 546 is shown as a single element, although it is understood that the second storage circuit 546 can be a distribution of storage elements. Also, for illustrative purposes, the compute system 100 is shown with the second storage circuit 546 as a single hierarchy storage system, although it is understood that the compute system 100 can include the second storage circuit 546 in a different configuration. For example, the second storage circuit 546 can be formed with different storage technologies forming a memory hierarchal system including different levels of caching, main memory, rotating media, or off-line storage.

The second storage circuit 546 can be a controller of a volatile memory, a nonvolatile memory, an internal memory, an external memory, or a combination thereof. For example, the second storage circuit 546 can be a controller of a nonvolatile storage such as non-volatile random-access memory (NVRAM), Flash memory, disk storage, or a volatile storage such as static random access memory (SRAM). The second storage circuit 546 can facilitate the sponsoring member 118 and the members 120 of the shared risk pool 122 to access the probational member status 109 and provide for the feedback 117 from the sponsoring member 118.

The second storage interface 548 can receive information from the other functional units/circuits or from external sources, or can transmit information to the other functional units/circuits or to external destinations. The external sources and the external destinations refer to sources and destinations external to the second device 106.

The second storage interface 548 can include different implementations depending on which functional units/circuits or external units/circuits are being interfaced with the second storage circuit 546. The second storage interface 548 can be implemented with technologies and techniques similar to the implementation of the second control interface 544.

The second communication circuit 536 can enable external communication to and from the second device 106. For example, the second communication circuit 536 can permit the second device 106 to communicate with the first device 102 over the network 104.

The second communication circuit 536 can also function as a communication hub allowing the second device 106 to function as part of the network 104 and not limited to be an endpoint or terminal unit or circuit to the network 104. The second communication circuit 536 can include active and passive circuitry components, such as microelectronics, wireless circuitry, wireline circuitry, analogue circuitry, or an antenna, for interaction with the network 104.

The second communication circuit 536 can include a second communication interface 550. The second communication interface 550 can be used for communication between the second communication circuit 536 and other functional units or circuits in the second device 106. The second communication interface 550 can receive information from the other functional units/circuits or can transmit information to the other functional units or circuits.

The second communication interface 550 can include different implementations depending on which functional units or circuits are being interfaced with the second communication circuit 536. The second communication interface 550 can be implemented with technologies and techniques similar to the implementation of the second control interface 544, including circuitry, waveguides, wireless circuitry, wireline circuitry, analogue circuitry, or a combination thereof.

The second communication circuit 536 can couple with the network 104 to send information to the first device 102, including the optimized parameters 122 in the second device transmission 510. The first device 102 can receive information in the first communication circuit 516 from the second device transmission 510 of the network 104. The compute system 100 can be executed by the first control circuit 512, the second control circuit 534, or a combination thereof. For illustrative purposes, the second device 106 is shown with the partition containing the second user interface 538, the second storage circuit 546, the second control circuit 534, and the second communication circuit 536, although it is understood that the second device 106 can include a different partition. For example, the second software 542 can be partitioned differently such that some or all of its function can be in the second control circuit 534 and the second communication circuit 536. Also, the second device 106 can include other functional units or circuits not shown in FIG. 5 for clarity.

The sponsoring member 118 can access the probational member status 109 for assisting the probational member 105 in qualifying to join the shared risk pool 122. Each of the members 120 of FIG. 1 of the shared risk pool 122 can access the probational member status 109 in preparation for voting on the acceptance of the probational member 105.

The functional units or circuits in the first device 102 can work individually and independently of the other functional units or circuits. The first device 102 can work individually and independently from the second device 106 and the network 104.

The functional units or circuits in the second device 106 can work individually and independently of the other functional units or circuits. The second device 106 can work individually and independently from the first device 102 and the network 104.

The functional units or circuits described above can be implemented in hardware. For example, one or more of the functional units or circuits can be implemented using a gate array, an application specific integrated circuit (ASIC), circuitry, a processor, a computer, integrated circuit, integrated circuit cores, a pressure sensor, an inertial sensor, a microelectromechanical system (MEMS), a passive device, a physical non-transitory memory medium containing instruction for performing the software function, a portion therein, or a combination thereof.

For illustrative purposes, the compute system 100 is described by operation of the first device 102 and the second device 106. It is understood that the first device 102 and the second device 106 can operate any of the modules and functions of the compute system 100.

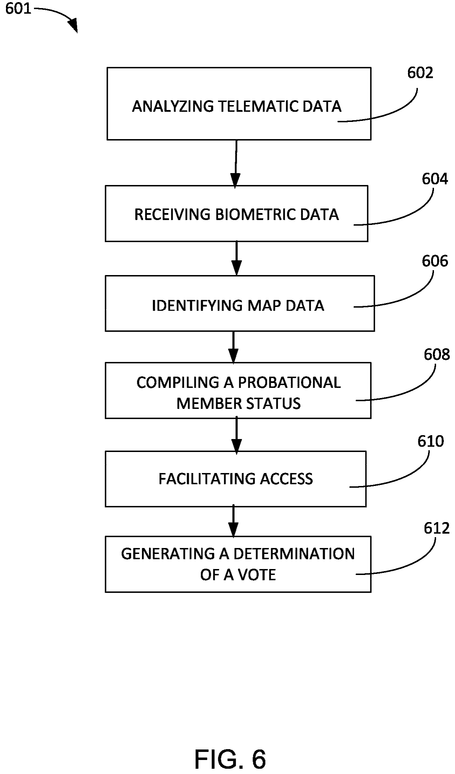

Referring now to FIG. 6, therein is shown a flow chart of a method 600 of operation of a compute system 100 of FIG. 1 in an embodiment of the present invention. The method 600 includes: analyzing telematics data of a first device operated by a probational member in a block 602; receiving biometric data of the probational member in a block 604; identifying map data based on the telematics data, the biometric data, or a combination thereof in a block 606; compiling a probational member status based on the telematics data, biometric data, the map data, or a combination thereof concurrently compiled by real-time monitoring a the probational member in a block 608; facilitating a sponsoring member and a members of a shared risk pool to access the probational member status in a block 610; and generating a determination of a vote by the sponsoring member and the member for an admission of the probational member to the shared risk pool based on the probational member status in a block 612.

The resulting method, process, apparatus, device, product, and/or system is straightforward, cost-effective, uncomplicated, highly versatile, accurate, sensitive, and effective, and can be implemented by adapting known components for ready, efficient, and economical manufacturing, application, and utilization. Another important aspect of an embodiment of the present invention is that it valuably supports and services the historical trend of reducing costs, simplifying systems, and increasing performance.

These and other valuable aspects of an embodiment of the present invention consequently further the state of the technology to at least the next level.

While the invention has been described in conjunction with a specific best mode, it is to be understood that many alternatives, modifications, and variations will be apparent to those skilled in the art in light of the foregoing description. Accordingly, it is intended to embrace all such alternatives, modifications, and variations that fall within the scope of the included claims. All matters set forth herein or shown in the accompanying drawings are to be interpreted in an illustrative and non-limiting sense.

## Claims

1. A compute system (100) comprising:
a control unit (113) configured to:
analyze telematics data (114) of a first device (102) operated by a probational member (105);
receive biometric data (116) of the probational member (105);
identify map data (112) based on the telematics data (114), the biometric data (116), or a combination thereof;
compile a probational member status (109) based on the telematics data (114), biometric data (116), the map data (112), or a combination thereof concurrently compiled by real-time monitoring the probational member (105);
facilitate a sponsoring member (118) and a member (120) of a shared risk pool (122) to access the probational member status (109); and
generate a determination of a vote (312) by the sponsoring member (118) and the member (120) for an admission of the probational member (105) to the shared risk pool (122) based on the probational member status (109).

2. The system (100) as claimed in claim 1 wherein the control unit (113) is configured to compile the probational member status (109) includes a telematics interface unit (114) coupled to the first device (102) to collect a performance characteristic (107) in real-time when the probational member (105) is driving the first device (102).

3. The system (100) as claimed in claim 1 or 2, wherein the control unit (113) is configured to compile the probational member status (109) includes a biometric monitor unit (116) coupled to the probational member (105) to collect a performance characteristic (107) in real-time for monitoring health of the probational member (105).

4. The system (100) as claimed in any one of claims 1-3 wherein the control unit (113) is configured to compile the probational member status (109) includes the control unit (113) coupled to a telematics interface unit (114) to monitor acceleration, braking, speed, swerving, use of lighting and turn signals by the probational member (105).

5. The system (100) as claimed in any one of claims 1-4 wherein the control unit (113) is configured to compile the probational member status (109) includes the control unit (113) coupled to a biometric monitor unit (116) to monitor body temperature, heart rate, breathing rate, oxygen saturation, and regularity of the heartbeat in real-time of the probational member (105).

6. The system (100) as claimed in any one of claims 1-5 further comprising a storage circuit (546) is coupled to a sponsor interface (119) to provide feedback (117) to the sponsoring member (118) for coaching the probational member (105).

7. The system (100) as claimed in claim 1-6 further comprising a storage circuit (546) is configured to facilitate the member (120) of the shared risk pool (122) to vote (312) on the probational member (105) to join the shared risk pool (122).

8. A method (600) of operation for a compute system (100) comprising:
analyzing telematics data (114) of a first device (102) operated by a probational member (105);
receiving biometric data (116) of the probational member (105);
identifying map data (112) based on the telematics data (114), biometric data (116), or a combination thereof;
compiling a probational member status (109) based on the telematics data (114), biometric data (116), the map data (112), or a combination thereof concurrently compiled by real-time monitoring of the probational member (105);
facilitating a sponsoring member (118) and a member (120) of a shared risk pool (122) to access the probational member status (109); and
generating a determination of a vote (312) by the sponsoring member (118) and the member (120) for an admission of the probational member (105) to the shared risk pool (122) based on the probational member status (109).

9. The method (600) as claimed in claim 8 further comprising compiling the probational member status (109) includes a telematics interface unit (114) coupled to the first device (102) to collect a performance characteristic (107) in real-time when the probational member (105) is driving the first device (102).

10. The method (700) as claimed in claim 8 or 9 further comprising compiling the probational member status (109) includes a biometric monitor unit (116) coupled to the probational member (105) to collect a performance characteristic (107) in real-time for monitoring health of the probational member (105).

11. The method (700) as claimed in any one of claims 8-10 further comprising compiling the probational member status (109) includes monitoring acceleration, braking, speed, swerving, use of lighting and turn signals of the first device (102) by the probational member (105).

12. The method (700) as claimed in any one of claims 8-11 further comprising compiling the probational member status (109) includes monitoring body temperature, heart rate, breathing rate, oxygen saturation, and regularity of the heartbeat in real-time of the probational member (105).

13. The method (700) as claimed in any one of claims 8-12 further comprising providing feedback (117) to the sponsoring member (118) for coaching the probational member (105).

14. The method (700) as claimed in any one of claims 8-13 further comprising facilitating the member (120) of the shared risk pool (122) to vote (312) on the probational member (105) to join the shared risk pool (122).

15. A non-transitory computer readable medium including instructions for a compute system (100) comprising:
analyzing telematics data (114) of a first device (102) operated by a probational member (105);
receiving biometric data (116) of the probational member (105);
identifying map data (112) based on the telematics data (114), biometric data (116), or a combination thereof;
compiling a probational member status (109) based on the telematics data (114), biometric data (116), the map data (112), or a combination thereof concurrently compiled by real-time monitoring of the probational member (105);
facilitating a sponsoring member (118) and a member (120) of a shared risk pool (122) to access the probational member status (109); and
generating a determination of a vote (312) by the sponsoring member (118) and the member (120) for an admission of the probational member (105) to the shared risk pool (122) based on the probational member status (109).
